# EUROPEAN PATENT SPECIFICATION

(11) **EP 4 303 814 B1**
(45) Date of publication and mention of the grant of the patent: **25.06.2025**
(21) Application number: 23183521.6
(22) Date of filing: 05.07.2023
(51) Int. Cl.: G06T 7/30

(54) **MEDICAL IMAGE REGISTRATION METHOD AND APPARATUS**
VERFAHREN UND VORRICHTUNG ZUR REGISTRIERUNG MEDIZINISCHER BILDER
PROCÉDÉ ET APPAREIL D'ENREGISTREMENT D'IMAGE MÉDICALE

(30) Priority: 08.07.2022 KR 20220084496
(43) Date of publication of application: 10.01.2024
(73) Proprietor: Medicalip Co., Ltd., Gangwon-do 24341 (KR)
(72) Inventor: KIM, Jong Min, Yongin-si (KR); SEDIQI, Khwaja Monib, Seoul (KR); PARK, Sang Joon, Seoul (KR)
(74) Representative: Patentanwälte Ruff, Wilhelm, Beier, Dauster & Partner mbB

(56) References cited:
- US-A1- 2022 012 927
- US-B1- 10 984 530
- BOVEIRI HAMID REZA ET AL: "Medical image registration using deep neural networks: A comprehensive review", COMPUTERS & ELECTRICAL ENGINEERING, PERGAMON PRESS, GB, vol. 87, 28 July 2020 (2020-07-28), XP086303385, ISSN: 0045-7906, [retrieved on 20200728], DOI: 10.1016/J.COMPELECENG.2020.106767
- YINGDA XIA ET AL: "Detecting Pancreatic Ductal Adenocarcinoma in Multi-phase CT Scans via Alignment Ensemble", ARXIV.ORG, CORNELL UNIVERSITY LIBRARY, 201 OLIN LIBRARY CORNELL UNIVERSITY ITHACA, NY 14853, 1 July 2020 (2020-07-01), XP081714690
- BOAH KIM ET AL: "CycleMorph: Cycle Consistent Unsupervised Deformable Image Registration", ARXIV.ORG, CORNELL UNIVERSITY LIBRARY, 201 OLIN LIBRARY CORNELL UNIVERSITY ITHACA, NY 14853, 13 August 2020 (2020-08-13), XP081739958

## Description

The invention relates to a method and apparatus for registering medical images, and more particularly, to a method and apparatus for registering medical images by using an artificial intelligence model.

The invention refers to a technology developed through the "Korea Data and Software-Driven Hospitals Consortium 2.0)" project supervised by Seoul National University Bundang Hospital (task number: S0252-21-1001). This application is based on and claims priority under 35 U.S.C. §119 to Korean Patent Application No. 10-2022-0084496, filed on July 8, 2022, in the Korean Intellectual Property Office, the disclosure of which is incorporated by reference herein in its entirety.

When computed tomography (CT) or magnetic resonance imaging (MRI) is performed, contrast agents are administered to a patient to make specific tissues, blood vessels, or lesions more visible. For example, an abdominal CT scan may be taken before injecting contrast agents into a patient, and an abdominal CT scan may be taken again after injecting contrast agents into the patient. Medical images such as CT images may be divided into a plurality of phases (e.g., pre, artery, post, delay, etc.) according to the degree of contrast enhancement after administration of the contrast agents. A medical staff may give a diagnosis by comparing medical images of different phases. For example, a medical staff may diagnose liver cancer based on the size and location of liver lesions appearing in medical images of various phases. However, when CT or MRI is performed, the position and size of the liver and lesion appearing on a medical image of each phase may be different due to the movement or breathing of a patient. In this case, the diagnosis accuracy is low.

Patent publication US 10,984,530 B1 discloses a medical image prcessing method and computing device which comprise acquiring series of enhanced medical images, detecting a phase of each enhanced medical image in the series of enhanced medical images using a pre-trained 3D convolutional neural network model, selecting a plurality of target enhanced medical images from all the enhanced medical images according to the phases, obtaining a plurality of interest images by segmenting an interest region in each of the plurality of target enhanced medical images, and registering the plurality of interest images.

It is the technical problem underlying the invention to provide a medical image registration method and apparatus which avoid or alleviate shortcomings of the prior art as well as a related computer-readable recording medium.

The invention solves this problem by providing a medical image registration method having the features of claim 1 and a medical image registration apparatus having the features of claim 5 as well as a computer-readable recording medium having the features of claim 7. Advantageous embodiments of the invention are mentioned in the dependent claims the wording of which is herewith incorporated into the description by reference to avoid unnecessary text repetitions.

One or more embodiments of the invention include a method and apparatus for registering medical images captured after administering a contrast agent to a patient.

Additional aspects will be set forth in part in the description which follows and, in part, will be apparent from the description, or may be learned by practice of the presented embodiments of the disclosure.

According to the invention, a medical image registration method includes receiving a medical image captured using a contrast agent, determining a phase of the medical image by using a classification model, and registering the medical image to a reference image by using a registration model, wherein the classification model includes an artificial intelligence model that outputs the phase of the medical image, and the registration model includes an artificial intelligence model for registering the medical image to the reference image.

According to the invention, a medical image registration apparatus includes an image input unit configured to receive a medical image captured using a contrast agent, a phase determination unit configured to determine a phase of the medical image by using a classification model, and a registration unit configured to register the medical image to a reference image by using a registration model, wherein the classification model includes an artificial intelligence model that outputs the phase of the medical image, and the registration model includes an artificial intelligence model for registering the medical image to the reference image.

The above and other aspects, features, and advantages of the invention will be more apparent from the following description of certain embodiments taken in conjunction with the accompanying drawings, in which:
FIG. 1 is a diagram illustrating examples of a phase of a medical image captured by administering a contrast agent to a patient;
FIG. 2 illustrates an example of a medical image registration apparatus according to an embodiment;
FIG. 3 illustrates an example of medical image according to an embodiment;
FIG. 4 illustrates an example of classification model according to an embodiment;
FIG. 5 illustrates an example of registration model according to an embodiment;
FIG. 6 illustrates an example of a plurality of registration models according to an embodiment;
FIG. 7 is a flowchart of an example of a medical image registration method according to an embodiment;
FIG. 8 illustrates a configuration of an example of a medical image registration apparatus according to an embodiment; and
FIG. 9 is a diagram illustrating an example of a result of image registration by using a medical image registration method according to an embodiment.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Hereinafter, a medical image registration method and apparatus according to an embodiment will be described in detail with reference to the attached drawings.

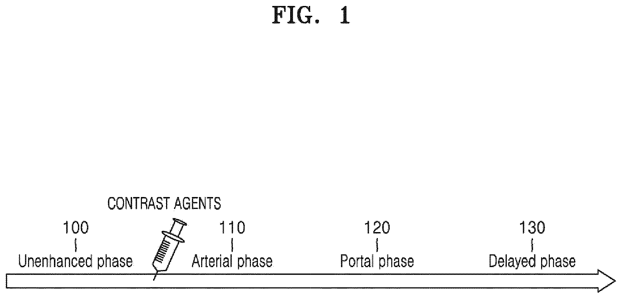

FIG. 1 is a diagram illustrating examples of a phase of a medical image captured by administering a contrast agent to a patient.

Referring to FIG. 1, a medical image may be captured by administering a contrast agent to a patient so that a specific tissue, blood vessel, or lesion is more visible. The medical image may be a computed tomography (CT) or magnetic resonance imaging (MRI) image, and hereinafter, for convenience of description, description will be based on a CT image.

The degree of contrast enhancement varies as time passes after a contrast agent is administered to a patient, and phases of medical images are divided according to the degree of contrast enhancement. For example, a medical image captured by administering a contrast agent may be classified into an arterial phase 110, a portal phase 120, and a delayed phase 130. A medical image captured before administration of a contrast agent may be classified as an unenhanced phase 100.

The present embodiment is only an example for explaining that the phases (100, 110, 120, 130) before and after the administration of a contrast agent may be divided into a plurality of sections according to the degree of contrast enhancement, and the number and type of phases may be set variously according to embodiments. For example, the portal phase 120 may be further subdivided into a plurality of sections, such as an early portal phase, a middle portal phase, and a late portal phase. However, hereinafter, for convenience of description, the phase of a medical image will be described with reference to FIG. 1.

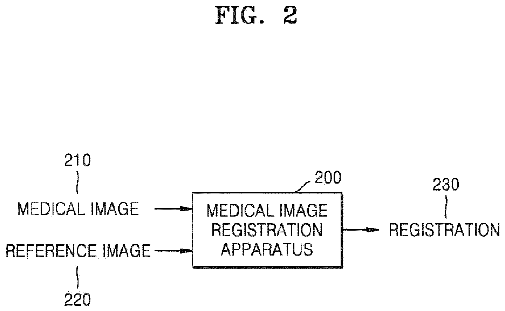

FIG. 2 illustrates an example of a medical image registration apparatus according to an embodiment.

Referring to FIG. 2, upon receiving a medical image 210 and a reference image 220, a medical image registration apparatus 200 registers the medical image 210 to the reference image 220 (230). The medical image 210 is an image to which movement, rotation, or resizing is applied for registration, and may be an image captured before administration of a contrast agent or an image captured after administration of a contrast agent. The reference image 220 is a fixed image and may be an image captured before administration of a contrast agent or an image captured after administration of a contrast agent.

When images of various phases are captured, the medical image 210 and the reference image 220 may be selected in various manners. For example, in the example of FIG. 1, the medical image registration apparatus 200 may select an image of the portal phase as the reference image 220 and receive an image of the arterial phase as the medical image 210.

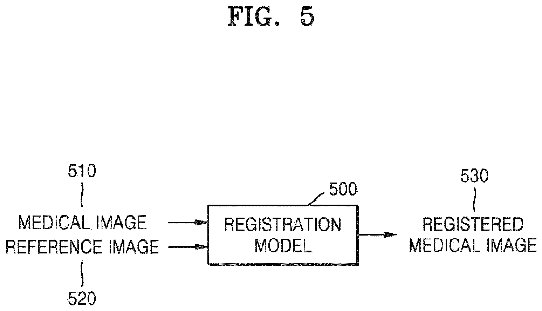

As described with reference to FIG. 1, in a medical image for each phase, the shapes of various tissues in an image appear differently according to the degree of contrast enhancement. Thus, if an image registration algorithm according to the related art is used as it is, registration accuracy may be low. For example, when a registration algorithm used to register two images of the arterial phase and the portal phase, respectively, is used to register the two images of the portal phase and the delayed phase, the registration accuracy may decrease. Accordingly, according to the present embodiment, a registration method using a plurality of registration models that perform registration between images of different phases is suggested. A registration model according to the present embodiment will be described again with reference to FIG. 5.

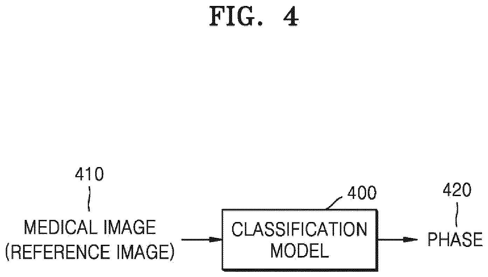

When registration between a plurality of different phases is performed using the registration model according to the present embodiment, it may be important to distinguish exactly of what phase the medical image 210 and the reference image 220 are. The degree of contrast enhancement changes over time after administration of a contrast agent, which may differ from patient to patient. For example, a first patient may reach the portal phase after passage of time t1 after administration of a contrast agent, and a second patient may reach the portal phase after passage of time t2. Thus, only accurate identification of the phase of a medical image allows use of an accurate registration model suitable for the phase. Accordingly, in the present embodiment, a classification model for determining a phase of a medical image is included, which will be described with reference to FIG. 4.

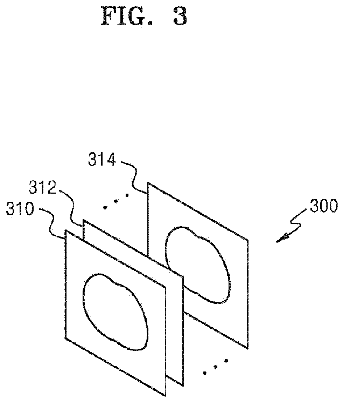

FIG. 3 illustrates an example of medical image according to an embodiment.

Referring to FIG. 3, a medical image 300 may include a plurality of cross-sectional images 310, 312, and 314. For example, when taking an abdominal CT scan of a patient, the medical image 300 may include the plurality of cross-sectional images 310, 312, and 314 captured at regular intervals. For example, a medical image of the arterial phase may be composed a plurality of cross-sectional images obtained by photographing the abdomen in the arterial phase at regular intervals after administration of a contrast agent.

When the medical image 300 includes the plurality of cross-sectional images 310, 312, and 314, registration of images of different phases may be a process of registration all of the plurality of cross-sectional images. For example, when a medical image is composed of N cross-sectional images of the arterial phase (where N is a natural number of 2 or more), and a reference image is composed of M cross-sectional images of the delayed phase (M may be the same as or different from N), the medical image registration apparatus may register all N cross-sectional images of the arterial phase to M cross-sectional images of the delayed phase.

According to the present embodiment, the medical image 300 including the plurality of cross-sectional images 310, 312, and 324 is suggested, but this is only an example, and the medical image and the reference image to be registered may also be each composed of a single cross-sectional image. However, hereinafter, for convenience of description, a case in which both the medical image and the reference image are composed of a plurality of cross-sectional images will be described.

FIG. 4 illustrates an example of classification model according to an embodiment.

Referring to FIG. 4, a classification model 400 is an artificial intelligence model that predicts and outputs a phase 420 upon receiving a medical image 410 (or a reference image). The classification model 400 may be implemented using various artificial neural networks such as a Convolutional Neural Network (CNN). For example, the classification model 400 may be implemented by a Visual Geometry Group (VGG).

The classification model 400 may be generated by training by using learning data obtained by labeling medical images of various phases, by phase. For example, the classification model 400 may be trained by a supervised method by using various medical images for the four phases of the unenhanced phase 100, the arterial phase 110, the portal phase 120, and the delayed phase 130, as learning data. Since the method of training and generating an artificial intelligence model using learning data is already a well-known method, additional description thereof will be omitted.

FIG. 5 illustrates an example of registration model according to an embodiment.

Referring to FIG. 5, a registration model 500 is an artificial intelligence model that outputs a registered medical image 530 when images of different phases are input. The registration model 500 may be implemented by various artificial neural networks such as CNN. The registration model 500 may be generated by training by using learning data including images of two types of phases (e.g., a medical image 510 and a reference image 520) and a registered medical image 530. For example, the registration model 500 that registers two medical images of a portal phase and a delayed phase may be generated using learning data including the two medical images of the portal phase and the delayed phase and a registration medical image of the two medical images of the portal phase and the delayed phase.

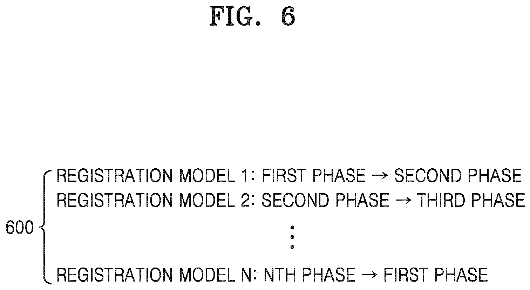

Upon receiving the medical image 510 and the reference image 520, the registration model 500 that has been trained moves, rotates, or resizes the medical image 510 such that the medical image 510 matches the reference image 520 and outputs a result. For example, if the phase of the medical image 510 is the portal phase and the phase of the reference image 520 is the delayed phase, the registration model 500 adjusts a position or size of the medical image 510 of the portal phase to match the reference image 520 and outputs a result. It is difficult for one registration model 500 to perform all registration between various phases. Accordingly, in the present embodiment, a plurality of registration models for registration between each phase is included, and an example thereof is shown in FIG. 6.

FIG. 6 illustrates an example of a plurality of registration models according to an embodiment.

Referring to FIG. 6, there are a plurality of registration models 600 for registration of images of different phases. For example, a first registration model may be a model that performs registration between images of a first phase and a second phase, and a second registration model may be a model that performs registration between images of the second phase and a third phase, and an Nth registration model may be a model that performs registration between images of the Nth phase and the first phase. The number of registration models may vary according to the number of phases. The plurality of registration models 600 may be generated by the method described with reference to FIG. 5.

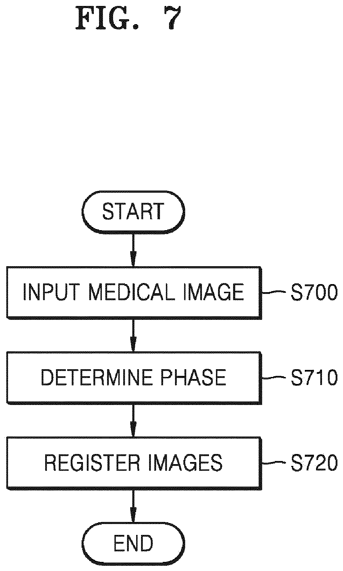

FIG. 7 is a flowchart of an example of a medical image registration method according to an embodiment.

Referring to FIG. 7, the medical image registration apparatus 200 receives a medical image in operation S700. The medical image may include a medical image captured using a contrast agent.

The medical image registration apparatus 200 determines a phase of the medical image in operation S710. For example, the medical image registration apparatus 200 may determine the phase of the medical image by using the classification model 400 of FIG. 4. As another embodiment, the medical image registration apparatus 200 may additionally determine a phase of a reference image. For example, when the exact phase of the reference image is not known, the medical image registration apparatus 200 may determine the phase of the reference image by using the classification model 400. If phase information of the reference image is known in advance, the determining of the phase of the reference image may be omitted.

The medical image registration apparatus 200 registers a medical image to a previously designated reference image by using a registration model in operation S720. When registration between a plurality of phases is required, there is the plurality of registration models 600 described with reference to FIG. 6. In this case, the medical image registration apparatus 200 selects a registration model to be used for registration, by using the phase of the medical image and the phase of the reference image. For example, in the example of FIG. 6, if the phase of the medical image is the second phase and the phase of the reference image is the third phase, the medical image registration apparatus performs registration between the medical image and the reference image by using the second registration model.

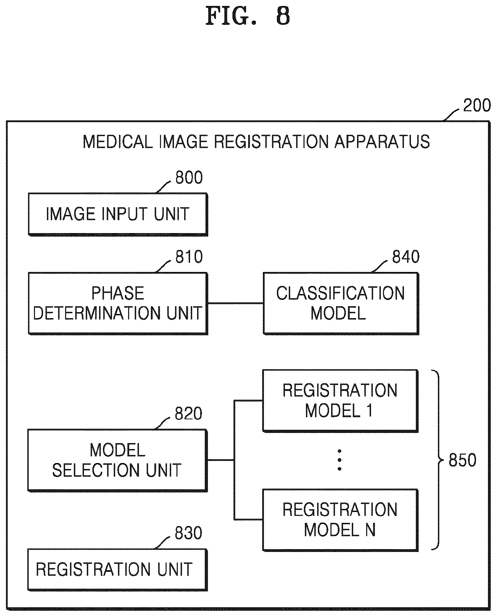

FIG. 8 illustrates a configuration of an example of a medical image registration apparatus according to an embodiment.

Referring to FIG. 8, the medical image registration apparatus 200 includes an image input unit 800, a phase determination unit 810, a model selection unit 820, a registration unit 830, a classification model 840, and at least one registration model 850. The medical image registration apparatus 200 may be implemented using a computing device including a memory, a processor, an input/output device, and the like. In this case, each component may be implemented as software and loaded in a memory, and then performed by a processor.

The image input unit 800 receives a medical image and a reference image. The medical image and the reference image may be three-dimensional medical images captured using a contrast agent.

The phase determination unit 810 determines a phase of the medical image by using a classification model. When a phase of the reference image is not known, the phase determination unit 810 may additionally determine the phase of the reference image by using a classification model.

The model selection unit 820 selects one registration model to be used for registration from among a plurality of registration models 850 based on the phase of the medical image and the phase of the reference image.

The registration unit 830 registers the medical image to the reference image by using the registration model.

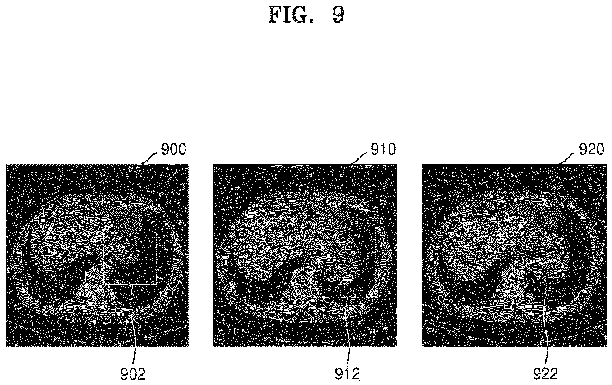

FIG. 9 is a diagram illustrating an example of a result of image registration by using a medical image registration method according to an embodiment.

Referring to FIG. 9, an image 920 is shown in which a medical image 900 of the delayed phase is modified to be registered to a reference image 910 of the portal phase by the medical image registration method according to the present embodiment. In the present embodiment, one image of each phase is shown for convenience of description, but an image of each phase may also be composed of a plurality of cross-sectional images as described with reference to FIG. 3.

As evident from square boxes 902, 912, and 922 of the images 900, 910, and 920, the medical image 920 after registration is accurately compared with the reference image 910 compared to the medical image 900 in the delay phase. For example, when using phase-specific medical images for liver cancer diagnosis, medical staff may more accurately compare the size and location of liver cancer, thereby increasing the accuracy of diagnosis.

The disclosure may also be implemented as computer-readable code on a computer-readable recording medium. The computer-readable recording medium includes all types of recording devices in which data readable by a computer system is stored. Examples of computer-readable recording media include read only memory (ROM), random access memory (RAM), a compact disk ROM (CD-ROM), a solid state disk (SSD), and optical data storage devices. In addition, the computer-readable recording medium is distributed in a network-connected computer system so that the computer-readable code can be stored and executed in a distributed manner.

While the disclosure has been particularly shown and described with reference to embodiments thereof, it will be understood by those of ordinary skill in the art that various changes in form and details may be made therein without departing from the scope of the disclosure as defined by the appended claims. The disclosed embodiments should be considered in a descriptive sense only and not for purposes of limitation. Therefore, the scope of the disclosure is defined not by the detailed description of the disclosure but by the appended claims, and all differences within the scope will be construed as being included in the disclosure.

According to an embodiment, the accuracy of medical image registration may be increased by using a registration model suitable for a phase of a medical image. In addition, the accuracy of diagnosis may be improved through registration of medical images of various phases. For example, the accuracy of liver cancer diagnosis may be increased by registering the size and location of liver lesions appearing in medical images of various phases.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope of the disclosure as defined by the following claims.

## Claims

1. Medical image registration method comprising:
- receiving a medical image captured using a contrast agent;
- determining a phase of the medical image by using a classification model which comprises an artificial intelligence model that outputs the phase of the medical image; and
- registering the medical image to a reference image by using a registration model which comprise an artificial intelligence model for registering the medical image to the reference image,
- wherein a plurality of registration models is provided for registering images of different phases and the registering comprises selecting one of the registration models to be used for registration, based on the phase of the medical image and a phase of the reference image.

2. Medical image registration method of claim 1, wherein each of the medical image and the reference image comprises a three-dimensional image including a plurality of cross-sectional images.

3. Medical image registration method of claim 1 or 2, wherein the medical image and the reference image each comprise a computed tomography (CT) image or a magnetic resonance imaging (MRI) image.

4. The medical image registration method of any of claims 1 to 3, further comprising determining the phase of the reference image by using the classification model.

5. Medical image registration apparatus comprising:
- an image input unit (800) configured to receive a medical image captured using a contrast agent;
- a phase determination unit (810) configured to determine a phase of the medical image by using a classification model (840);
- a registration unit (830) configured to register the medical image to a reference image by using a registration model (850); and
- a model selection unit (820) configured to select one registration model to be used for registration from among a plurality of registration models provided for registering images of different phases, based on the phase of the medical image and a phase of the reference image,
- wherein the classification model (840) comprises an artificial intelligence model that outputs the phase of the medical image, and
- the registration model comprises an artificial intelligence model for registering the medical image to the reference image.

6. Medical image registration apparatus of claim 5, wherein the phase determination unit is further configured to determine the phase of the reference image by using the classification model.

7. Computer-readable recording medium having recorded thereon a computer program for performing the method according to any of claims 1 to 4.

## Patentansprüche

1. Medizinbildregistrierungsverfahren, umfassend:
- Empfangen eines unter Verwendung eines Kontrastmittels aufgenommenen medizinischen Bildes;
- Bestimmen einer Phase des medizinischen Bildes unter Verwendung eines Klassifikationsmodelles, das ein Modell mit künstlicher Intelligenz beinhaltet, das die Phase des medizinischen Bildes ausgibt; und
- Registrieren des medizinischen Bildes zu einem Referenzbild unter Verwendung eines Registrierungsmodells, dass ein Modell mit künstlicher Intelligenz zum Registrieren des medizinischen Bildes zu dem Referenzbild beinhaltet;
- wobei eine Mehrzahl von Registrierungsmodellen zum Registrieren von Bildern unterschiedlicher Phasen vorgesehen ist und das Registrieren ein Auswählen eines für die Registrierung zu benutzenden Registrierungsmodells basierend auf der Phase des medizinischen Bildes und einer Phase des Referenzbildes umfasst.

2. Medizinbildregistrierungsverfahren nach Anspruch 1, wobei jedes von dem medizinischen Bild und dem Referenzbild ein dreidimensionales Bild mit einer Mehrzahl von Querschnittbildern umfasst.

3. Medizinbildregistrierungsverfahren nach Anspruch 1 oder 2, wobei das medizinische Bild und das Referenzbild jeweils ein Computertomographie(CT)-Bild oder ein Magnetresonanzbildgebungs(MRI)-Bild beinhaltet.

4. Medizinbildregistrierungsverfahren nach einem der Ansprüche 1 bis 3, dass des Weiteren ein Bestimmen der Phase des Referenzbildes unter Verwendung des Klassifikationsmodells umfasst.

5. Medizinbildregistrierungsvorrichtung mit:
- einer Bildeingabeeinheit (800), die zum Empfangen eines unter Verwendung eines Kontrastmittels aufgenommenen medizinischen Bildes eingerichtet ist;
- einer Phasenbestimmungseinheit (810), die zum Bestimmen einer Phase des medizinischen Bildes unter Verwendung eines Klassifikationsmodells (840) eingerichtet ist;
- einer Registrierungseinheit (830), die zum Registrieren des medizinischen Bildes zu einem Referenzbild unter Verwendung eines Registrierungsmodells (850) eingerichtet ist; und
- einer Modellauswahleinheit (820), die zum Auswählen eines für die Registrierung zu benutzenden Registrierungsmodells aus einer Mehrzahl von zum Registrieren von Bildern verschiedener Phasen bereitgestellten Registrierungsmodellen basierend auf der Phase des medizinischen Bildes und einer Phase des Referenzbildes eingerichtet ist;
- wobei das Klassifikationsmodell (840) ein Modell mit künstlicher Intelligenz umfasst, das die Phase des medizinischen Bildes ausgibt, und
- das Registrierungsmodell ein Modell mit künstlicher Intelligenz zum Registrieren des medizinischen Bildes zu dem Referenzbild umfasst.

6. Medizinbildregistrierungsvorrichtung nach Anspruch 5, wobei die Phasenbestimmungseinheit des Weiteren dafür eingerichtet ist, die Phase des Referenzbildes unter Verwendung des Klassifikationsmodells zu bestimmen.

7. Computerlesbares Aufzeichnungsmedium, auf dem ein Computerprogramm zur Durchführung des Verfahrens nach einem der Ansprüche 1 bis 4 aufgezeichnet ist.

## Revendications

1. Méthode d'enregistrement d'images médicales comprenant:
- la réception d'une image médicale capturée à l'aide d'un agent de contraste;
- déterminer une phase de l'image médicale à l'aide d'un modèle de classification comprenant un modèle d'intelligence artificielle qui produit la phase de l'image médicale; et
- enregistrer l'image médicale sur une image de référence en utilisant un modèle d'enregistrement qui comprend un modèle d'intelligence artificielle pour enregistrer l'image médicale sur l'image de référence,
- dans laquelle une pluralité de modèles d'enregistrement est prévu et l'enregistrement consiste à sélectionner l'un des modèles d'enregistrement à utiliser pour l'enregistrement, sur la base de la phase de l'image médicale et d'une phase de l'image de référence.

2. Méthode d'enregistrement d'images médicales de la revendication 1, dans laquelle l'image médicale et l'image de référence comprennent chacune une image tridimensionnelle incluant une pluralité d'images transversales.

3. Méthode d'enregistrement d'images médicales de la revendication 1 ou 2, dans laquelle l'image médicale et l'image de référence comprennent chacune une image de tomographie assistée par ordinateur (CT) ou une image d'imagerie par résonance magnétique (MRI).

4. La méthode d'enregistrement d'images médicales de l'une des revendications 1 à 3, comprenant en outre la détermination de la phase de l'image de référence à l'aide du modèle de classification.

5. Appareil d'enregistrement d'images médicales comprenant
- une unité d'entrée d'image (800) configurée pour recevoir une image médicale capturée à l'aide d'un agent de contraste;
- une unité de détermination de la phase (810) configurée pour déterminer la phase de l'image médicale à l'aide d'un modèle de classification (840);
- une unité d'enregistrement (830) configurée pour enregistrer l'image médicale à une image de référence en utilisant un modèle d'enregistrement (850); et
- une unité de sélection de modèle (820) configurée pour sélectionner un modèle d'enregistrement à utiliser pour l'enregistrement parmi une pluralité de modèles d'enregistrement fournis pour enregistrer des images de phases différentes, sur la base de la phase de l'image médicale et d'une phase de l'image de reference;
- dans lequel le modèle de classification (840) comprend un modèle d'intelligence artificielle qui produit la phase de l'image médicale, et
- le modèle d'enregistrement comprend un modèle d'intelligence artificielle permettant d'enregistrer l'image médicale sur l'image de référence.

6. Appareil d'enregistrement d'images médicales de la revendication 5, dans lequel l'unité de détermination de phase est en outre configurée pour déterminer la phase de l'image de référence en utilisant le modèle de classification.

7. Support d'enregistrement lisible par ordinateur sur lequel est enregistré un programme informatique pour l'exécution de la méthode selon l'une des revendications 1 à 4.
